# EUROPEAN PATENT APPLICATION

(11) **EP 4 566 624 A1**
(43) Date of publication of application: **11.06.2025**
(21) Application number: 23213860.2
(22) Date of filing: 04.12.2023
(51) Int. Cl.: A61K 39/002, A61P 33/02

(54) **VACCINES AGAINST PROTOZOAN PARASITES**

(71) Applicant: VacciMed-Oral GmbH, 53175 Bonn (DE)
(72) Inventor: Lujan, Hugo, 53175 Bonn (DE)
(74) Representative: Kröncke, Rolf

(57) **Abstract**

In a first aspect, the present invention relates to vaccines for preventing or treating protozoan parasite infections in an individual whereby the vaccine contains multiple protozoan variable surface antigens (VSAs) of said protozoan parasites microorganism. In particular, protective vaccines are disclosed. In a further aspect, a method for the preparation of the vaccine, in particular, the protective vaccine is described. The vaccine is composed of multiple distinguishable VSAs, preferably comprising the full repertoire of the different VSA encoded by the gene family in said protozoan parasite. In a second aspect, the present invention relates to vaccines for preventing or treating microbial infections in an individual whereby the vaccine contains microvesicles induced by confronting protozoan microorganisms with antibodies directed to their VSAs. In particular, protective vaccines are disclosed. The vaccine is composed of multiple antigens of said protozoan parasite. In a further aspect, methods for the preparation and administration of these vaccines, in particular, the protective vaccines are described.

## Description

In a first aspect, the present invention relates to vaccines for preventing or treating protozoan parasite infections in an individual whereby the vaccine contains multiple protozoan variable surface antigens (VSAs) of said protozoan parasites microorganism. In particular, protective vaccines are disclosed. In a further aspect, a method for the preparation of the vaccine, in particular, the protective vaccine is described. The vaccines are composed of multiple distinguishable VSAs, preferably comprising the full repertoire of the different VSA encoded by the gene family in said protozoan parasite. In a second aspect, the present invention relates to vaccines for preventing or treating microbial infections in an individual whereby the vaccine contains microvesicles induced by confronting protozoan microorganisms with antibodies directed to their VSAs. In particular, protective vaccines are disclosed. The vaccines may further contain multiple antigens of said protozoan parasites apart from the VSAs. In a further aspect, methods for the preparation and administration of these vaccines, in particular, the protective vaccines are described.

### Prior Art

A characteristic developed by parasitic organisms is their great capacity to adapt to environmental changes. Most parasites, whether unicellular such as protozoa or multicellular such as helminths, occupy different niches during their passage through vectors and hosts and have evolved extraordinary defense mechanisms that allow them to survive in environmental conditions that would otherwise destroy them. Thus, the survival of pathogenic microorganisms depends not only on their ability to colonize a host but also on their ability to counteract its defense mechanisms. Thus, the pathogenicity or virulence of parasites reflects the dynamic interaction between them and the host and their capacity to respond to the defensive systems of the infected individual, a necessary condition for parasitic survival and the maintenance and/or transmission of the infection. Remarkably, some parasites have developed mechanisms of evasion of the immune response (i. e., antigenic variation/serotype replacement), which allows them to survive and complete their life cycle within the host (Zambrano-Vila S, et. al., Trends Parasitol. 2002;18(6):272-8).

Antigenic variation is a clonal phenotypic variation developed by some pathogenic microorganisms involving surface-exposed antigenic determinants. These organisms use different mechanisms to change the expression of their surface antigens to maintain chronic infection under continuous immune pressure generated by their host. Antigenic variation in unicellular microorganisms involves three essential requirements: (i) a large family of homologous genes encoding immunodominant surface antigens; (ii) a mechanism that allows the exclusive expression of a single member of that family on individual cells; and (iii) a molecular process that allows the change of the originally expressed antigen to another of the same family (Deitsch KW, et. al., Nat Rev Microbiol [Internet]. 2009; 7:493-503. Available from: http://dx.doi.org/10.1038/nrmicro2145).

With respect to antigenic variation, many parasitic microorganisms are protected by a tight coat of molecules called variable surface proteins/glycoproteins/adhesins (VSAs, also identified as VSPs or VSGs, in the following generally referred to as VSAs), which cover the entire surface of the parasite and towards which the host immune response is generated. Each individual microorganism expresses only one VSA from a repertoire of dozens to thousands of VSAs at any given time and, for reasons not well known, this antigen is changed to another antigenically different. Thus, the host immune response is evaded and chronic and/or recurrent infections are generated.

To date, the only possibility of controlling most parasitic diseases is to use drugs whose active ingredients are not efficient, present many side effects, and have been reported to have resistant strains. In addition, for parasites that manifest antigenic variation, re-infection is frequent after treatment because these individuals do not generate efficient defense mechanisms to eliminate the parasite. In developing countries, the continued mortality and morbidity caused by infections are an indication that ongoing treatments are inadequate.

In the genome of the protozoan parasites, there are a multiplicity of genes encoding variable surface antigens, but only one is expressed at any given time on the surface of each cell. From this, it is easy to conclude that to counteract this mechanism of parasite evasion the host should generate antibodies directed against each of these antigens. Methodologically this is impossible, but what can be done, thanks to the studies carried out in recent years, is to stimulate the mechanism of antigenic variation of the parasite in such a way that it can be "forced" to express the entire repertoire of its variable antigens. Thus, it would be possible to immunize individuals using these cells as a vaccine and thus generate in the host a specific and protective immune response directed against the entire repertoire of potential surface antigens. In the event of subsequent reinfections, the immune system will respond rapidly to these trophozoites thanks to the memory immune response.

It was recently determined that low concentrations of antibodies directed against variable surface antigens of known isolates of *Giardia lamblia* (Variant-specific Surface proteins or VSPs), *Trypanosoma brucei* (Variant Surface Glycoproteins or VSGs), and even of the free-living protozoan *Tetrahymena thermophila* (i-Antigens) induce antigenic switching. Indeed, incubation of clones of these protozoa with antibodies directed against their corresponding variable antigen generated parasite populations expressing multiple variants, and extracellular microvesicles containing the antibody target antigen were generated during this process (https: //www. biorxiv.org/content/10.1101/2022.06.21.497077v1). Therefore, these populations could become a fundamental tool to generate vaccines against protozoan parasites for which antigenic variation has been demonstrated but also for others in which this phenomenon has not been yet reported *(Trypanosoma cruzi*/*Trichomonas vaginalis*/*Tritrichomonas foetus).* Unfortunately, the reports on the stimulation of antigenic variation in protozoa were only based on the description of the molecular and structural processes of the antibody/variable surface antigen interaction and did not consider that they could be used in formulations that induce a protective response against infections by these microorganisms and that, at present, there are still no vaccines against parasites that manifest antigenic variation.

The flagellate protozoan *Giardia lamblia* (syn. G. *duodenalis* or G. *intestinalis)* is one of the most common causes of human intestinal disease worldwide and one of the most frequent enteric pathogens of domestic and farm animals, as it is possibly the only microorganism capable of colonizing both the duodenum and jejunum, a highly hostile environment where nutrient digestion takes place. Infection is initiated by ingestion of cysts of the parasite usually present in contaminated water or food. *Giardia* has been reported as the cause of outbreaks of waterborne diarrhea in developed countries, as well as in cases of diarrhea in daycare centers, institutionalized persons, and travelers. In Asia, Africa, and Latin America, about 200 million people suffer from symptomatic giardiasis, and about 500,000 new cases are reported each year. In regions where basic sanitation is poor, *Giardia* infections are almost universal by two years of age. *Giardia* has been recognized as a re-emerging infection and included in the "Neglected Diseases Initiative" by WHO. In addition, clinical manifestations of giardiasis, such as diarrhea, anorexia, weight loss, and lethargy, have been associated with giardiasis in both domestic and farm animals. Some antiparasitic drugs are commonly used to treat giardiasis in animals and humans, but reinfections are frequent and drug-resistant strains have already been described.

*Giardia* has a simple life cycle consisting of an infective cyst and a vegetative trophozoite. Infection is transmitted by ingestion of cysts, which are excreted in the feces. After excystation in the upper small intestine, flagellated trophozoites are released. Trophozoites are non-invasive, proliferate attached to the surface of intestinal epithelial cells by a sucking disc, but do not invade the mucosa and cause little or no mucosal inflammation. Trophozoites are responsible for the clinical manifestations associated with the disease, which range from asymptomatic infections to severe acute or chronic diarrhea. Some individuals are chronically infected without symptoms of diarrhea, but suffer malabsorption and, predominantly in young individuals, long-term detrimental effects on growth and development. The immune status of the host influences its susceptibility to infection and the severity of clinical signs. Young or elderly individuals are especially susceptible to Giardia infections. Symptoms usually appear one to two weeks after infection and last 2 to 5 days. Giardiasis appears to be limited, but in some cases, chronic infections occur in the absence of any apparent immunodeficiency.

The prevalence of *Giardia* has been studied and isolates obtained from different hosts were characterized at the molecular level to determine their zoonotic potential (Sprong H, et. al., PLoS Negl Trop Dis. 2009;3(12):1-12). Phylogenetic analyses identified eight assemblages (genetic groups) in *G. lamblia* that were named A to H. It was observed that assemblages A and B could infect several mammals and may become reservoirs for human infections, just as humans may also be a potential reservoir of *Giardia* for domestic and production animals. *G. canis* (assemblage C), *G. cati* (assemblage F), and G. *bovis* (assemblage E) would be limited to certain species or host types, whereas other *Giardia* assemblages have a wide host range, including assemblages A and B that infect humans and can be transmitted to livestock and domestic animals (Thompson RCA, et. al., Parasitol Today. 2000;16(5):210-3 (11-13)).

Antigenic variation in *Giardia* involves Variant-specific Surface Proteins (VSPs). VSPs are integral cysteine-rich membrane proteins that possess a variable extracellular N-terminal region (ectodomain), a conserved C-terminal region that includes a single transmembrane domain, and a short cytoplasmic tail of only five amino acids. The ectodomain of VSPs allows the parasite to survive in the upper portion of the small intestine because these proteins are highly resistant to pH changes (between pH 1 and 10) and to degradation by trypsin and other proteases present in the gastrointestinal tract. VSPs form a thick layer over the parasite that represents the host-parasite interface. Only one VSP, out of a repertoire of approximately 140 homologous genes present in the parasite genome, is expressed on the surface of each trophozoite at any given time (Nash TE., Philos Trans R Soc B Biol Sci. 1997;352:1369-75), but the switch to the expression of an antigenically distinct VSP occurs even in the absence of any immune pressure, albeit with a very low turnover frequency. Both posttranscriptional and epigenetic mechanisms have been reported to regulate the expression of a single variant in each trophozoite (Prucca CG, et. al., Nature. 2008; 456(7223):750-4). Since disruption of the mechanism of antigenic variation has previously been shown to be crucial for generating effective vaccines (Rivero FD, et. al., Nat Med [Internet]. 2010;16(5):551-7. Available from: http://dx.doi.org/10.1038/nm.2141), the utilization of *Giardia* populations containing trophozoites expressing the complete repertoire of VSPs resistant to proteolytic degradation would allow their use as a vaccine formulation to be administered orally to generate a mucosal immune response that protects against subsequent infections. Recently it has been demonstrated that this antigenic variation can be induced by the presence of antibodies against the VSP-expressing clonal population producing the replacement of the original PSV by a multiplicity of VSPs in trophozoites of the resulting population. In addition, it was verified that the original VSP was released into the extracellular medium included in microvesicles while the switching to other VSPs was taking place.

Human African Trypanosomiasis or Sleeping Sickness is a vector-borne parasitosis. It is caused by *Trypanosoma brucei,* a protozoan transmitted by the bite of the tsetse fly. It is an infection that, without treatment, is lethal. This parasitosis is endemic in 36 countries in sub-Saharan Africa, where tsetse flies, specifically of the *Glossina spp.* transmitting the disease, are present.

Human African Trypanosomiasis caused devastating epidemics during the 20th century. But thanks to consistent and coordinated efforts in recent years, the number of reported cases has declined ("WHO Human African trypanosomiasis" 2022). The disease remains endemic in parts of sub-Saharan Africa, where it represents a major problem for rural communities, particularly in central Africa. As cases have also been reported in patients from non-endemic countries, this disease should be considered in the differential diagnosis of travelers, tourists, emigrants, and expatriates who have visited or lived in endemic areas. Population displacement, war and poverty are important factors facilitating transmission. The drugs available for treatment are not optimal; in the absence of a vaccine, disease control relies on case detection and treatment, as well as vector control.

*Trypanosoma brucei* is a hemoflagellate protozoan of the genus Trypanosoma. Two subspecies that are morphologically indistinguishable cause distinct disease patterns in humans: *T. b. gambiense,* which causes chronic African Trypanosomiasis, and *T. b. rhodesiense,* which causes the acute form. The third subspecies *T. b. brucei* is a parasite mainly of cattle and occasionally other animals, and under normal conditions does not infect humans, although the presence of trypanosomiasis in domestic animals, particularly cattle, is a major obstacle to the economic development of affected rural areas (https://www.cdc.gov/dpdx /trypanosomiasisafrican/index.html).

*T. brucei* is an extracellular parasite that undergoes several developmental transformations both in the blood of the mammalian host and in different compartments of the female tsetse fly. When the parasite is ingested by the tsetse fly from an infected host, the intestinal conditions of the insect allow its morphological change to the procyclic form, followed by the epimastigote form and, finally, the infective metacyclic form in the salivary glands of the vector.

An infected tsetse fly injects metacyclic trypomastigotes into the skin tissue of a mammalian host. The parasites enter the lymphatic system and pass into the bloodstream. Within the host's bloodstream, they transform into trypomastigotes which are transported to other sites in the body, thus reaching other body fluids (lymph, cerebrospinal fluid) and continuing replication by binary fission.

Initially, the parasites multiply in the subcutaneous tissues, blood, and lymph, known as the endolymphatic phase, characterized by episodes of fever, headache, lymphadenopathy, joint pain, and pruritus. When the parasites cross the blood-brain barrier and infect the central nervous system, this is known as the neurological or meningoencephalic phase. This is usually when the most obvious signs and symptoms of the disease (behavioral changes, confusion, sensory disturbances and lack of coordination) occur. Sleep cycle disorders, which give the disease its name, are evident at this stage. If untreated, the disease is considered fatal, although cases of healthy carriers have been reported.

The type of treatment depends on the stage of the disease, as the earlier it is diagnosed, the better the chances of a cure. The success of second-stage treatment depends on a drug that crosses the blood-brain barrier to reach the parasite. In the first stage of the disease, pentamidine and suramin are recommended for T. *b. gambiense* and *T. b. rhodesiense* infections, respectively. Melarsoprol, a highly toxic arsenic derivative, is used for advanced stages of *T. b. rhodesiense* infections. Other treatments, such as eflornithine or the combination of nifurtimox-eflornithine (NECT) and, more recently, fexinidazole (first oral treatment), are more commonly used in chronic disease. Unfortunately, these drugs are highly toxic and resistant strains have been found (Fall F, et. al., Metabolomics [Internet]. 2022;18(4). Available from: https://doi.org/10.1007/s11306-022-01880-0).

The presence of regularly changing environments requires microbial pathogens to adapt rapidly to fluctuating conditions. Like other parasites, *Trypanosoma brucei* encounters different environments as it transitions between its mammalian host and vector. In these different environments, the parasite has had to evolve to optimize its survival and ensure transmission to the next host (Silvester E, et. al., PLoS Negl Trop Dis. 2018;12(10):1-25).

*Trypanosoma brucei* regularly changes its main surface antigen, variable surface glycoprotein (VSG), to evade the host immune response. Antigenic variation is a key pathogenic mechanism that allows *T. brucei* to establish long-term infections under continuous immune pressure generated by its host. VSGs are a family of highly immunogenic proteins (Silva-barrios S, et. al., TrendsParasitol 2017;34(2):1-17. Available from: http://dx.doi.org/10.1016/j.pt.2017.10.001). However, *T. brucei* undergoes antigenic variations and sequentially expresses immunologically distinct VSGs, effectively evading the host response. VSG expression is important for parasite virulence.

Trichomoniasis caused by *Tritrichomonas foetus* is a sexually transmitted disease listed by the World Organization for Animal Health (OIE). This disease is one of the main causes of early reproductive failure in cattle, causing important economic losses. It is currently considered an endemic disease affecting herds managed under grazing conditions with natural service breeding and where artificial insemination is included as a method for reproduction.

Recently, *T. foetus* has been shown to colonize portions of the large intestine of cats and other felids producing chronic, recurrent diarrhea with mucus and fresh blood, often unresponsive to common drugs. Without proper treatment, the disease may resolve spontaneously in months or years, but cats may continue to carry the parasite.

These microorganisms reside in the epithelium of the preputial cavity of infected bulls; the epithelial crypts of the foreskin provide a microaerophilic environment that favors the replication of these parasites. Consequently, bulls can develop a lifelong infection without showing symptoms of disease.

*Trichomonas* have been shown to adhere to sperm causing decreased sperm motility, agglutination, and phagocytosis, and can survive even during semen cryopreservation. Transmission of *T. foetus* to the female can cause vaginitis, cervicitis, endometritis, infertility, delayed return to estrus, low pregnancy rate, early embryonic death and even abortions. It has been demonstrated that *T. foetus* can be invasive for the fetus, having been found in the placenta, fetal lung, intestine, and lymph nodes.

*Tritrichomonas foetus* is perhaps the non-human *Trichomonas* more similar to *Trichomonas vaginalis.* Besides having three anterior flagella compared to the four of *T. vaginalis,* there are few morphological differences between the two parasites. Both *T. foetus* and *T. vaginalis* contain gene families in their genomes that code for adhesins and variable surface antigens (Burgess DE, et. al., Infect Immun. 1990;58(11):3627-32).

*T. foetus* is pyriform in shape, has a single nucleus, an undulating membrane with three to five waves and a characteristic vibratory motion, which gives it a distinctive diagnostic feature when comparing *T. foetus* with most other protozoa found in preputial samples contaminated with fecal material.

The life cycle is simple since it only presents the trophozoite stage, although in recent years a form called pseudocyst has been described. Some authors argue that, under well-controlled experimental conditions in vitro, as well as in vivo in infected bulls, the parasite acquires a spherical or elliptical shape, and the flagella are internalized, but the cells do not show an encysted wall. This form, known as endo flagellar or pseudo cystic, is viable and can revert to pear-shaped trophozoites.

The drug of choice for the treatment of bovine trichomoniasis is Metronidazole. The treatment has been associated with a temporary resolution of clinical signs but is not able to control the disease. In recent years, the emergence of aerobic and anaerobic resistance *in vivo* and *in vitro* has been described as leading to ineffective treatments, (Rivero MB, et. al., Acta Parasitol 2019;64(2):232-5. Available from: https://doi.org/10.2478/s11686-019-00031-1).

The cellular mechanisms by which *T. foetus* colonizes mucosal surfaces and causes tissue damage are not well defined. Little is known about the mechanisms involved in the host-host relationship, as well as how the parasite evades the immune pressure generated by the host. For a better understanding of the biology and biochemistry of this protozoan, several authors have proposed various animal models that have helped to better understand this disease.

Some studies using Balb/c mice demonstrated that a vaginal infection with *T. foetus* could be established with only 100 trophozoites and that it could persist for more than 30 days, and even no pretreatment prior to inoculation was necessary for the establishment of the disease. In studies of *T. foetus* infection during early pregnancy using animal models, changes were found in the local immune response at the maternal-fetal interface, as well as in the expression of uterine epithelial carbohydrates. Some studies in these models led to the conclusion that induction of apoptosis is an additional mechanism involved in the pathogenesis of early embryonic death that occurs during infection with *T. foetus,* with increased cell proliferation being only a compensatory host response.

As it is evident from the above, the development of effective vaccines against protozoan parasites that undergo antigenic variation would lead to avoiding infection in hosts and limit the spread of the disease. However, the reality is that, despite the number of years that researchers have been studying the possibility of implementing new vaccines, there are few commercial vaccines against parasites.

### Brief description of the present invention

The present inventors aimed to provide suitable vaccines against protozoan parasites that undergo antigenic variation allowing in particular protective vaccination of individuals.

In a first aspect, the present invention relates to vaccines for preventing or treating protozoan parasite infections in an individual said vaccine comprising an active agent, and excipients, diluents or carriers and, optionally, adjuvants, wherein the active agent comprises multiple variable surface antigens (VSAs) of the parasite to be treated or to be protected against whereby the multitude of VSAs of the parasites are obtained by induction of expressing of multiple VSAs in a parasite clone expressing a single VSA whereby said clone was grown in the presence of a specific antibody against the single VSA present on the surface of said clone to induce the switching of VSA and the expression of multiple distinguishable VSAs in the population obtained by said culturing the clone. In a further aspect, the vaccine formulations include additionally microvesicles generated during the induction of antigenic variation containing parasite antigens. In particular, protective vaccines are provided.

In a further aspect, the present invention relates to a method for producing a vaccine according the present invention comprising the steps of:
- Providing a protozoan parasite clone expressing a single type of variable surface antigen (VSA) encoded in the genome of said protozoan parasite;
- Culturing said protozoan parasite clone in the presence of a specific concentration of an antibody against the single type of VSA for inducing antigenic variation;
- Propagation of the cultured and switched protozoan parasites expressing multiple VSAs within the population;
- Obtaining multiple VSAs in a form applicable in a vaccine;
- Optionally, obtaining microvesicles in a form of an applicable vaccine;
- Preparing the vaccine including mixing the multiple VSAs and, optionally the microvesicles, with excipients, diluents, or carriers.

### Brief description of the drawings

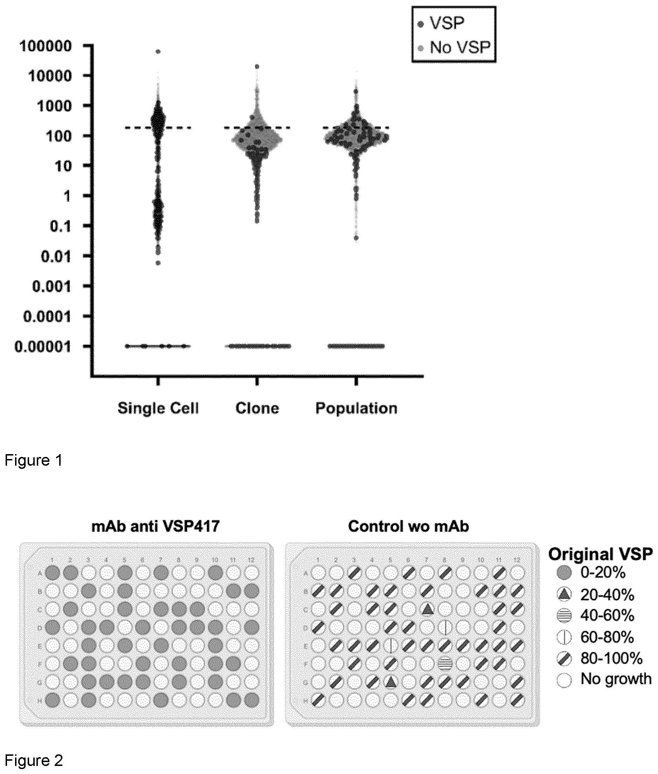

**Fig. 1****. Examples of VSP expression in RNAseq experiments.** Sequencing experiments of total mRNA extracted from Giardia trophozoites of all parasite genes (gray dots) except VSPs mRNAs (red dots): left; Experiment of individual trophozoites (Single Cell) expressing VSP417. Center; clonal population (Clone) of trophozoites expressing VSP417; Right; a Giardia population of a clone expressing VSP417 treated with the anti-VSP417 mAb 7C2 (Population). Individual cells and clones expressing VSP417 show high levels of VSP417 mRNA expression/accumulation (TPM values), which are then expressed on the surface of trophozoites. In contrast, in a non-clonal population, many VSPs are expressed at the level of most housekeeping genes (Population).
**Fig. 2****. Switching of single cells in culture in the presence of anti-VSP417 mAb** vs. clonality in single cell culture by limiting dilution with an unrelated mAb as control (mAb 8F12). The percentage of wells where parasites grew and the percentage of positivity of the original VSP from the preceding figure is shown.
**Fig. 3****. Formation of microvesicles containing the original antigen.** (top) TEM with gold particle-bound antibody labeling of a trophozoite expressing VSP417 incubated for 30 min with mAb 7C2 (black dots). (bottom) SEM of a VSP417-expressing trophozoite incubated with gold-labeled mAb 7C2 for 30 min showing an image obtained by backscattered electrons (yellow dots).
**Fig. 4****. Infection during vaccination.** Cysts per gram of fecal material collected from gerbils and hamsters vaccinated vs. non-vaccinated with assembly A (isolate WB) and assembly B (isolate GS).
**Fig. 5****. Infections during vaccination.** Cysts per gram of fecal material collected from vaccinated vs. non-vaccinated dogs and cats with isolate A (isolate WB) and isolate B (isolate GS).
**Fig. 6****. Infections in vaccinated animals.** Parasites per milliliter of blood collected from Balb/c mice vaccinated with microvesicles, vaccinated with inactivated parasites and unvaccinated animals (Control).
**Fig. 7****. Protection from infection in vaccinated animals.** The number of infected and non-infected animals according to control and vaccinated groups.

### Detailed description of the present invention

In a first aspect, the present invention relates to vaccines for preventing or treating protozoan parasite infections in an individual said vaccine comprising an active agent, and excipients, diluents or carriers and, optionally, adjuvants, wherein the active agent comprises multiple different variable surface antigens (VSAs) and, optionally microvesicles of the parasite to be treated or to be protecting against whereby the multitude of VSAs of the parasites and microvesicles are obtained by induction of antigenic variation in a parasite clone expressing a single VSA whereby said clone was grown in the presence of a specific antibody against the single VSA present on the surface of said clone to induce the switching of VSA to the expression of multiple distinguishable VSAs in the population obtained by said culturing of the clone.

As used herein, the term "multiple variable surface antigens" refers to a multitude of proteins/glycoproteins that can be expressed on the surface of parasites such as *Giardia lamblia* (VSP, variable surface proteins); *Trypanosoma brucei* (VSG, variant surface glycoproteins; *Trypanosoma cruzi* (transialidases), *Trichomonas vaginalis* and *T. foetus* (adhesins), etc. In an embodiment of the present invention, the multiple VSAs represent the full repertoire of VSAs encoded in the genome of the protozoan parasite.

The term "switching the variant of surface antigen" refers to the activation and process of the antigenic variation machinery as described above.

The term "microvesicles" refers to extracellular vesicles which size ranges between 60 and 200 nm that are released by incubating a parasite clone with antibodies directed to its VSA. Typically, these microvesicles are isolated from the supernatant of the culture by known methods. In an embodiment, these microvesicles comprise the VSA originally expressed by the cultured clone before switching. Further, other antigens stemming from the parasite are present.

The term "protective vaccine" refers to a vaccine that avoid infections in the host.

By starting the switching process of the VSA in a single clone of the protozoan parasite, it is possible to obtain successors of the single clone expressing multiple different VSAs and, in addition, microvesicles containing further multiple protective antigens can be obtained. This population of protozoan parasite stemming from the single clone can be obtained by incubation of the clone with a specific concentration of an antibody against the single VSA present on the surface of the single clone. This methodology allows the generation of populations of any parasite that can be cloned and induced to express the multiple different VSA, preferably, the full repertoire of potential VSA which in turn allow to provide an active agent of a vaccine suitable for the treatment of protozoan parasite infection and, moreover, for protective vaccination against subsequent infection with the protozoan parasite.

In an embodiment, the vaccine according to the present invention is a protective vaccine against subsequent infection with the protozoan parasite. As demonstrated, protection against subsequent infection can be achieved. It was not obvious from the art that the vaccine according to the present invention achieve protection of the vaccinated individual.

Further, in an embodiment of the present invention, the vaccine is against a protozoan parasite are selected from the genus of *Trypanosoma, Tritrichomonas, or Giardia.*

Moreover, in an embodiment of the present invention, the vaccine is against the protozoan parasite is selected from *Trypanosoma brucei, Tritrichomonas foetus, Giardia lamblia.*

Other protozoan parasites with VSAs can be used to generate a protective vaccination with the vaccine according to the present invention.

The individual to be vaccinated is in an embodiment a mammal including domestic or farm animal, or a human. The individual is typically the natural host of the protozoan parasite.

The active agent included in the vaccine according to the present invention may be an active agent provided in a form selected from: attenuated parasites, death parasites, parts of the parasites including plasma membrane, purified VSAs, microvesicles containing parasite antigens, or combinations thereof. The skilled person is well aware of suitable methods for preparing the active agent with known methods. Suitable methods are described in the examples below.

In an embodiment, the vaccine comprises as active agents the multiple distinguishable VSAs and the microvesicles. The components may be present as a mixture or as separate components which may allow administration simultaneously, separately or sequentially.

The vaccines may be adapted for mucosal and/or systemic administration. That is, the skilled person may provide the vaccine in a from for administration depending on the protozoan parasite and its place in the body of the individual where can be infected with the protozoan parasites.

Further, the vaccines may be adapted for administration by the mucosal route, like the intranasal route, or orally. Alternatively, the administration may be conducted by intravenous administration, intramuscular administration, or subcutaneous administration.

The vaccines may contain additionally suitable diluents, carrier or excipients or effluents. The skilled person is well aware of the same, in particular, depending on the way of administration.

The vaccines may contain additionally one or more types of adjuvants. Adjuvants in vaccine are known to the skilled person. The term "adjuvant" means substances which are added and/or co-formulated in a vaccine or in immunization to an active agent, i.e., the substance which provokes the desired immune response, in order to enhance or elicit or modulate the humoral and/or cell-mediated (cellular) immune response against the active agent. Preferably, the adjuvant can enhance or elicit the immune response. The skilled person is aware of suitable adjuvants for administration in vaccination strategies.

The vaccines according to the present invention contains the active agent in a therapeutically or pharmaceutically effective amount. That is, the amount is sufficient to induce the desired result, the vaccination, like protective vaccination.

The term "administered" means administration of a therapeutically effective dose of the vaccine to an individual, that is, a dose that produces the effects for which it is administered.

The exact dose will depend on the purpose of the treatment, and will be ascertainable by one skilled in the art.

In an embodiment, the vaccine according to the present invention comprise administration of two doses of VSAs in an amount of 50 to 500, like 200 µg per g body weight within an interval of 10 to 20 days, like 15 days. Of course, the exact dosage depends on various parameters of the individual to be vaccinated.

In an embodiment of the present invention, the vaccine is provided in a formulation of a topical creme for administering multiple times, in particular, 15 days for two months. In another embodiment, the vaccine being a protective vaccine for administration by food or drinking water of the mammal to be treated.

In another aspect of the present invention, a method for producing a vaccine according to the present invention is provided comprising the steps of:
- Providing a protozoan parasite clone expressing a single type of multiple variable surface antigens (VSA) encoded in the genome of said protozoan parasite clone;
- Providing protozoan microvesicles obtained from incubating the parasite clone with antibodies to its expressed VSA.
- Culturing said protozoan parasite clone in the presence of a specific antibody against the single type of VSA for inducing the switching of the VSA;
- Propagation of the cultured and switched protozoan parasite expressing multiple VSAs;
- Obtaining multiple VSAs in a form applicable in a vaccine;
- Optionally obtaining microvesicles in form applicable in a vaccine; and
- Preparing the vaccine including mixing with excipients, diluents, or carriers.

In an embodiment, the specific antibody against the single type of VSA is a monoclonal antibody. Of course, the antibody may be a polyclonal antibody. Any type of molecule binding specifically to the single type of VSA expressed initially by the protozoan parasite clone may be applied unless the molecule induces a switching resulting in antigenic variation.

Further, the specific concentration of the antibody can be easily determined by the skilled person by routine measures.

In an embodiment, in the method for producing the vaccine according to the present invention the step of obtaining the multiple VSA in a form applicable in a vaccine includes the purification and/or the inactivation of the protozoan parasite after cultivation and propagation. Further, microvesicles, if present in the vaccine according to the present invention and obtained in the method according to the present invention whereby the microvesicles are released into the culture medium during cultivation and propagation can be purified by centrifugation and filtration, e.g. as described in the Examples. E.g., parasites that have modified their surface antigen and microvesicles can be inactivated by treatment with β-propiolactone (1/1000 v/v), gamma irradiated (Furuya Y, et. al., J Virol. 2010;84(9):4212-21) or killed by sonication, or treated with 10 cycles of freezing at -180°C and rapid thawing at 37°C. In an embodiment, the present invention relates to vaccines against *Trypanosoma brucei* and related species and methods of manufacture for use in mammals.

The vaccines can be administered intramuscularly or subcutaneously to provide protection at the systemic level.

The vaccines consist of an effective amount of an immunostimulatory mixture containing parasites induced by expressing their full repertoire of variable surface glycoproteins or microvesicles containing parasite antigens.

One method of the invention consists in administering a suspension of dead or inactivated parasites which were previously subjected to the process of induction of the turnover of their surface proteins.

Another method of the invention consists in administering a suspension of microvesicles generated when the parasites were previously subjected to the process of induction of switching of their VSAs.

In an embodiment, blood pleomorphic forms from a *T. brucei* clone are grown in a culture medium and in the presence of a specific antibody against their corresponding variable surface glycoprotein (VSG), which induces VSG exchange and the expression of multiple distinct VSGs in parasites of the resulting population, in addition to the release of microvesicles containing vital parasite antigens. *Trypanosoma brucei* clones that have modified their surface antigen and the released microvesicles can be collected by centrifugation and can be inactivated by treatment with β-propiolactone (1/1000 v/v), gamma irradiated (Furuya Y, et. al., J Virol. 2010;84(9):4212-21) or killed by sonication, or treated with 10 cycles of freezing at -180°C and rapid thawing at 37°C.

In an embodiment, the present invention relates to a vaccine against *Giardia lamblia* and a method of manufacture for use in domestic and farm animals. The vaccine can be administered orally, preferably in both domestic and farm mammalian animals, to provide systemic and mucosal protection.

The vaccine consists of an effective amount of an immunostimulant mixture containing parasites induced to express their full repertoire of variable surface proteins and microvesicles containing other parasite antigens.

The method of the invention consists in administering a suspension of dead or inactivated parasites which were previously subjected to the process of induction of switching of their surface proteins and a suspension of microvesicles generated when the parasites were induced to antigenic exchange.

Trophozoites from a *G. lamblia* clone are grown in a culture medium in the presence of a specific antibody against a variable surface protein (VSP) which induces the turnover of this VSP and the expression of multiple distinct VSPs in parasites of the resulting population, in addition to the release of microvesicles containing parasite antigens.

Trophozoites that have modified their surface antigen and the released microvesicles can be collected by centrifugation and can be inactivated by treatment with β-propiolactone (1/1000 v/v), gamma ray-irradiated, killed by sonication, or treated with 10 cycles of freezing at -180°C and rapid thawing at 37°C.

It is administered orally 2×10⁶ trophozoites per kilogram of body weight resuspended in 1 ml of sterile saline solution in two doses with an interval of 15 days to achieve systemic and mucosal protection in animals. This formulation can also be included in capsules that can be degraded in the intestine.

In an embodiment, the present invention relates to a vaccine against *Tritrichomonas foetus* and a method of manufacture for use in farm and domestic animals.

The vaccine can be administered orally or topically, preferably in farm and domestic mammalian animals, to provide protection at the mucosal level.

The vaccine consists of an effective amount of an immunostimulatory mixture containing parasites induced by expressing their full repertoire of VSA adhesins and variable surface antigens.

The method of the invention consists in administering a suspension of killed or inactivated parasites that were previously subjected to the induction of switching of their surface proteins. Optionally, microvesicles as described herein are administered simultaneously, separately or sequentially.

Trophozoites from a *T. foetus* clone are grown in a culture medium and in the presence of a polyclonal antibody which induces switching for the expression of multiple different surface proteins.

Trophozoites of *Tritrichomonas foetus* that have modified their surface antigen and the released microvesicles can be collected by centrifugation and can be inactivated by treatment with β-propiolactone (1/1000 v/v), gamma irradiated (Furuya Y, et. al., J Virol. 2010;84(9):4212-21) or killed by sonication, or treated with 10 cycles of freezing at -180°C and rapid thawing at 37°C.

Two doses of 2x106 trophozoites per kilogram of body weight resuspended in 1 ml of sterile saline solution are administered orally in two doses at an interval of 15 days to achieve systemic and mucosal protection in animals. It is also possible to include the formulation in topical creams to be administered every 15 days for two months.

Finally, the present invention relates to a method of prophylactic or therapeutic treatment of protozoan parasite infection in an individual. Said method includes the administration of the vaccine according to the present invention at least once to the individual. Administration may be affected once or several times.

The present invention will be described further by way of examples without limiting the same thereto.

### Examples

### Example 1

### 1. Giardia culture

*Giardia lamblia* trophozoites assemblage A1 isolate WB (ATCC@ 50803) and clone GS/M-83 (ATCC@ 50581) (assemblage B) were cultured in borosilicate glass tubes containing TYI-S-33 medium supplemented with 0.5 mg/ml bovine bile (Sigma-Aldrich, Cat. #B3883) and 10% adult bovine serum (Natocor) at 37°C. Clones expressing different VSPs were obtained by limiting dilution in 96-well culture plates placed in anaerobic chambers (AnaerogenTM Compact, Thermo Scientific^{®} Oxoid^{®}, Cat. no. AN0010C) at 37°C for 5 days and then positive clones were selected using anti-VSPs specific mAb by immunofluorescence assay (IFA). Reactive clones were expanded in a culture medium overnight and checked for homogeneity before use. For antigenic variation induction studies, cultures expressing a given VSP were recloned but in the presence of 50 nM of their specific anti-VSP mAb.

### 2. Laboratory animals

Balb/c mice, gerbils, and Syrian hamsters (6-8 weeks old) of both sexes were used and maintained under specific pathogen-free (SPF) conditions in boxes connected to microisolators, following NIH guidelines for laboratory animals.

### 3. Production of monoclonal antibodies

Monoclonal antibodies (mAbs) against individual VSPs were generated in 6-week-old Balb/c mice that were immunized subcutaneously with 200 µl of an HPLC-purified preparation of antigen emulsified with adjuvant (SigmaTM Adjuvant System MPL+TDM+CWS) according to the manufacturer's recommendation. Mice were immunized again after 21 days with 200 µl of the same preparation and 20 days later immunized intravenously with 100 µl with the same preparation. After three days, the mice were sacrificed, and the spleen cells were used for fusion to NSO myeloma cells. The antibody-secreting hybridomas were tested by ELISA assays and by immunofluorescence on fixed and permeabilized trophozoites. Purification of monoclonal antibodies was performed by FPLC using the ÄKTA Pure 25 apparatus (GE Healthcare Life Sciences) coupled to a HiTrapTM Protein G HP (GE Healthcare^{®}, Cat. # 29-0485¬81), following the manufacturer's protocol. After purification, a buffer change to PBS was performed using the same equipment coupled to the HiPrepTM 26/10 desalting column (GE Healthcare, Cat. # 17-5087-01). Subsequently, protein concentration was measured using the BCA Protein Assay Kit (Pierce^{®}, Cat. # 23225). The concentrations, expressed in molarity, are derived from the concentration of protein present in the purified antibodies, considering a molecular weight of 150 kDa (for IgG). The purified antibodies were stored in small aliquots at -20°C.

### 4. RNAseq analysis in populations

Total RNA was purified from the trophozoite population of assemblage A1 isolate WB (ATCC@ 50803) by extraction with Trizol following the manufacturer's protocol. Ribosomal RNA was removed from all samples and sequencing gen libraries were constructed by Illumina strand-specific sequencing. RNA sequencing was performed using a paid service from Genehub.com. The gen libraries were sequenced as 2x150 bp paired-end reads on an Illumina MiSeq, and 3.1¬4.3 million reads were subsequently generated for each library. The genome index was generated using RSubread with default parameters. Quality control of the raw reads was performed with FastQC v0.11.5. If necessary, low-quality bases (below 30) and adapter sequences were removed with TrimGalore v0.6.4 and Cutadapt v1.15 to improve mapping efficiency. Sequences shorter than 70 bp were removed. Reads that passed the quality criteria were aligned to the most recent RSubread reference genome with default parameters except for (i) phredOffset=33, (ii) unique = FALSE, (iii) nBestLocations = 2, which was set to 2 to account for duplicate genes. On average, -80% of all sequenced reads could be mapped to a single location in the genome. After mapping, reads were assigned to features using FeatureCounts in RSubread. Reads with multiple overlaps or those that did not overlap with any feature in the annotation file were not counted (Rodriguez-Walker M, et. al., Genomics. 2022;114(5), Liao Y, et. al., Nucleic Acids Res. 2019;47(8)).

### 5. Single-cell antigenic variation assay

Fifty trophozoites expressing a specific VSP (VSP417) were diluted in 20 ml of TYI-S-33 medium containing 50 nM of the corresponding anti-VSP mAb. They were distributed in 96-well plates. The same procedure was followed in the TYI-S-33 medium with an unrelated mAb as a control (mAb 8F12; anti-CWP2). The plates were incubated for 5 days; the number of clones obtained was then counted and the percentage of the original VSPs was measured by IFA.

### 6. Purification of microvesicles

Exponentially growing trophozoites (1.5x108) of clone VSP417 were washed twice with filtered PBS at 37°C and incubated for 4 h at 37°C in the presence of mAb 7C2 (50 nM) in microvesicle purification medium (ultrafiltered TYI-S-33 medium, 100 kDa MWCO, supplemented with 3% adult bovine serum previously ultracentrifuged overnight at 250,000 g). The tubes were cooled on ice and then centrifuged in an SW41Ti rotor (Beckman) at 4°C for 10 min. The supernatants were collected in 50 ml centrifuge tubes and centrifuged at 3,000 g at 4°C for 40 min; this step was repeated once. The supernatants were then concentrated 10X in a centrifugal filtration device (CentriconTM Plus-70-100K, Millipore^{®}, Cat. # UFC710008) and ultracentrifuged in a Beckman^{®} SW41Ti rotor (25,000 rpm, 2 h, at 4°C). The pellets were washed in an equal amount of ice-cold filtered PBS and ultracentrifuged again to recover the microvesicles. The purification of the microvesicles was validated by IFA.

### 7. Scanning electron microscopy (SEM) and helium ion microscopy (HM).

Glutaraldehyde-fixed cells were post-fixed in 1 % OsO4 for 15 min. The samples were then dehydrated in increasing series of ethanol up to 100 %, dried at the critical point with liquid CO2, and coated with charcoal to observe the cell surface in detail. The samples were examined on a QuantaTM SEM (FEI Co., The Netherlands) equipped with FEG filament. Images were obtained by secondary electron (SE) and backscattered electron (BSE) detection at an accelerating voltage of 15 kV. For high-resolution scanning microscopy analysis, the sputter coating step was performed using a thin (2 nm) platinum layer, and the cells were observed on an AurigaTM high-resolution SEM (Zeiss). For HM, cells were processed as described above and observed, without any subsequent coating, under a Zeiss Orion helium ion microscope.

### 8. Transmission electron microscopy (TEM).

Cells fixed with glutaraldehyde were subsequently fixed with 1 % OsO4 and 0.8 % potassium ferrocyanide for 40 min. Samples were dehydrated in increasing degrees of acetone to 100% and embedded in epoxy resin. Ultra-thin sections (50-60 nm thick) were cut, collected, and stained with uranyl acetate and lead citrate. Finally, the samples were analyzed with a TecnaiTM Spirit TEM (FEI Co.).

### 9. Infections with G. lamblia in gerbils and hamsters

Prior to infection, 6-week-old gerbils (Meriones unguiculatus) and Golden Hamsters (Mesocricetus auratus) were tested for the negativity of serum antibodies against *Giardia* antigens by ELISA. Ten animals of each species were inoculated for the trophozoite- and microvesicle-infected groups and 10 animals were used for each control group. Animals were immunized by orogastric administration of 2×10⁵ trophozoites and their microvesicles, which were resuspended in 300 µL of sterile PBS, with an interval of 15 days. Trophozoites were previously induced to switch by culturing in the presence of the specific mAb against the VSP expressed on the surface of the WB clone VSP417 (assemblage A) and GS VSPH7 (assemblage B) against which the microvesicles containing the membrane antigen of the original clone were released. These parasites were inactivated by sonication on ice (Al-Sabi MNS et. al., J Appl Microbiol. 2015;119(3):894-903). The absence of live parasites in the preparation was verified by culture in *Giardia* basic medium for up to one week after inoculation and by negativity to fluorescein diacetate staining (Saruyama N, et. al., Anal Biochem [Internet]. 2013;441(1):58-62. Available from: http://dx.doi.org/10.1016/j.ab.2013.06.005). Control animals were inoculated with 0.5 ml of PBS/cysteine by the same route. In all cases, vaccination did not trigger any signs of disease. After 2 months, the gerbil and hamster groups were inoculated with 1×10² cysts resuspended in 0.5 ml PBS with 5 mM cysteine. Fecal samples were collected from both groups of animals every three days between days 0 and 60 post-infection. Excreted cysts were visually identified by immunofluorescence assays using mAbs that recognize specific proteins in the Giardia cyst wall.

### 10. Protection for dogs and cats

Assays 6-8-week-old mongrel puppies and 5-8-week-old kittens were used. The animals were free of Giardia, which was determined by analysis of their feces by immunofluorescence assays using specific mAbs against cyst wall proteins, as well as of any other detectable infectious disease (all animals were fully vaccinated against common bacterial and viral infections before the start of the experimental period: Nobivac DP and Nobivac HHPPI from MSD Animal Health, Bagovac Rabies from Biogenesis-Bago). The animals were kept isolated in individual roofed compartments with access to an outdoor concrete-floored runway for immunizations and infections during the first months of the challenge experiments. They were offered autoclaved food and sterile water supplemented with a vitamin mixture (Vigorex, Labyes) ad libitum. All animals were housed following the standard operating procedures of the International Association for Laboratory Animal Care. Dogs and cats were tested for the presence of the parasite and treated with antiparasitic drugs prior to participation in the trial. Each animal was identified with a unique tag. Prior to infection, serum from all animals was tested for antibodies to Giardia antigens by enzyme-linked immunosorbent assay using a preparation of total proteins extracted from trophozoites and cysts. Animals with anti-Giardia antibodies were not included. Some animals were treated orally with 25 mg metronidazole and 25 mg albendazole per kg for 3 days, 10 days before testing, to rule out any possible presence of Giardia. Three days before day 0, the animals were weighed and randomly assigned to experimental (n=10) or control (n=10) groups, although the animals were housed in isolation from each other. The animals were clinically examined weekly throughout the trial. After the animals were infected, fecal samples were collected to detect the presence of Giardia cysts.

Animals were immunized with two oral administrations of 1 ml of parasites expressing the complete repertoire of VSPs (2×10⁶ trophozoites per kilogram weight) and their microvesicles resuspended in 0.5 ml of PBS, with an interval of 15 days that were previously induced to switch when cultured in the presence of the specific mAb against the VSP expressed on the surface of the original clone WB VSP417 (assemblage A) and GS VSPH7 (assemblage B) against including the microvesicles containing the membrane antigen of the original clone were released. The parasites were inactivated by sonication on ice (Al-Sabi MNS et. al., J Appl Microbiol. 2015;119(3):894-903). The absence of live parasites in the preparation was verified by culture in *Giardia* basic medium for up to one week after inoculation and by negativity to fluorescein diacetate staining (Saruyama N, et. al., Anal Biochem [Internet]. 2013;441(1):58-62. Available from: http://dx.doi.org/10.1016/j.ab.2013.06.005). Control animals were inoculated with 0.5 ml of PBS/cysteine by the same route.

Sixty days post immunization both groups were infected by orogastric inoculation of 1×102 cysts resuspended in 0.5 ml PBS with 5 mM cysteine. Freshly collected cysts from experimentally infected gerbils were used to prevent samples from rapidly losing viability and infectivity. Fecal samples were collected from both groups of animals every three days between days 0 and 60 post-infection, and weekly until day 120. Excreted cysts were visually identified by immunofluorescence assays using mAbs that recognize specific proteins in the Giardia lamblia cyst wall.

### Results

It was observed in a transcriptome of a *Giardia lamblia* clone of assembly A1 isolate WB (ATCC@ 50803) that the entire VSP repertoire is expressed with some level of detection but that the transcript that is translated and expressed on the surface of trophozoites is expressed/accumulated several orders of magnitude above any other gene. When individual cells were analyzed, the same was observed, as can be seen in clonal populations. On the other hand, clones expressing VSP417 that were incubated for six days with the anti-VSP417 antibody show the expression of multiple VSPs in that population (Table 1, Figure 1, and Table 2).

Table 1 shows the expression of selected VSP and Housekeeping genes in different *Giardia* transcriptomic experiments. Three single cells, three clones expressing VSP417, and three populations obtained after 6 days of culture in the presence of mAb 7C2 of VSP417-expressing cells were sequenced and analyzed. The values are expressed in TPM. A massive level of expression/accumulation of a particular VSP (highlighted in bold) could be observed both in single cells and in clonal populations (VSP GL50803-50229 and GL50803-d50229 are duplicated VSP genes). In contrast, no VSP has mRNA levels (highlighted in bold) well above any other Giardia gene in populations expressing multiple VSPs.

At the protein level, it was observed that when a clonal population of trophozoites expressing VSP417, VSP1267, or VSPH7 was cultured in 12-well plates in the continuous presence of their corresponding anti-VSP mAb (0.25-10 nM), after 6 days, in each well the surface antigen expression was modified, expressing in all cases VSPs different from the original, but not in the control experiment, where the rate of variation never exceeded 10% in that given period (Table 2).

**Table 2. Induction of switchers. Percentage of switchers after culturing a clonal population of VSP417, VSP1267 and VSPH7 trophozoites with their respective specific mAb and without antibodies.**

| | | mAb | | | Control | | |
|---|---|---|---|---|---|---|---|
| | | | | | | | |
| Culture # | Well # | VSP417 Switchers | VSP1267 Switchers | VSPH7 Switchers | VSP417 Switchers | VSP1267 Switchers | VSPH7 Switchers |
| 1 | 1 | 98% | 99% | 97% | 8% | 0% | 2% |
| | 2 | 98% | 98% | 100% | 3% | 0% | 9% |
| | 3 | 99% | 99% | 97% | 10% | 0% | 6% |
| | 4 | 98% | 98% | 98% | 10% | 0% | 8% |
| | 5 | 99% | 98% | 100% | 7% | 1% | 2% |
| | 6 | 99% | 100% | 97% | 3% | 1% | 7% |
| | 7 | 98% | 98% | 100% | 1% | 1% | 4% |
| | 8 | 99% | 99% | 98% | 5% | 1% | 0% |
| | 9 | 98% | 98% | 99% | 4% | 1% | 7% |
| | 10 | 100% | 99% | 98% | 0% | 1% | 8% |
| | 11 | 98% | 99% | 100% | 4% | 1% | 6% |
| | 12 | 98% | 98% | 100% | 9% | 1% | 5% |
| 2 | 1 | 98% | 98% | 99% | 3% | 0% | 6% |
| | 2 | 99% | 98% | 99% | 7% | 0% | 1% |
| | 3 | 99% | 98% | 99% | 2% | 0% | 6% |
| | 4 | 99% | 99% | 99% | 7% | 0% | 7% |
| | 5 | 98% | 99% | 100% | 0% | 1% | 1% |
| | 6 | 100% | 99% | 97% | 9% | 1% | 3% |
| | 7 | 99% | 100% | 98% | 9% | 1% | 2% |
| | 8 | 98% | 100% | 99% | 3% | 1% | 8% |
| | 9 | 99% | 99% | 98% | 2% | 1% | 7% |
| | 10 | 99% | 99% | 99% | 10% | 1% | 1% |
| | 11 | 98% | 100% | 99% | 4% | 1% | 3% |
| | 12 | 100% | 98% | 100% | 7% | 1% | 2% |

The same was observed when trophozoites from a clonal population expressing VSP417 were distributed in 96-well plates by limiting dilution. In the plate in which trophozoites were cultured with their corresponding mAb, after 6 days, in the wells where there was growth, the original VSP was not found, whereas in the control plate and in the presence of an unrelated antibody, the trophozoites were found to express the original VSP in a high proportion (Figure 2).

To determine the turnover of the original VSP after treatment with the antibody against the corresponding VSP, transmission (TEM) and scanning (SEM) microscopy analyses were performed using antibodies labeled with gold particles.

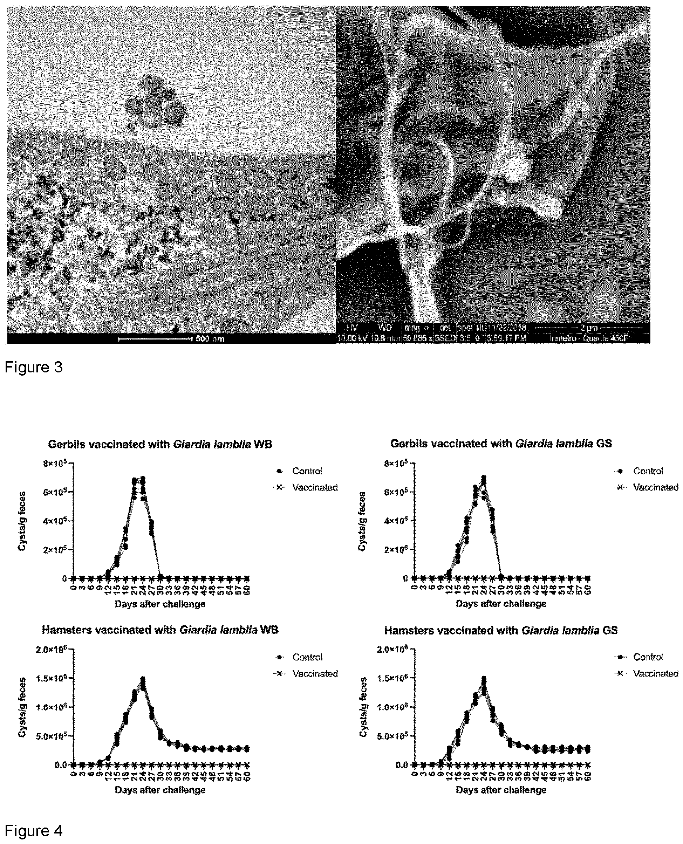

The results showed accumulations of microvesicles containing on their surface the VSP target of the corresponding antibody (Figure 3).

To determine whether these trophozoites and their microvesicles stimulated the mucosal immune system, especially secretory immunoglobulin A (slgA), immunizations and challenges were performed in various animal models.

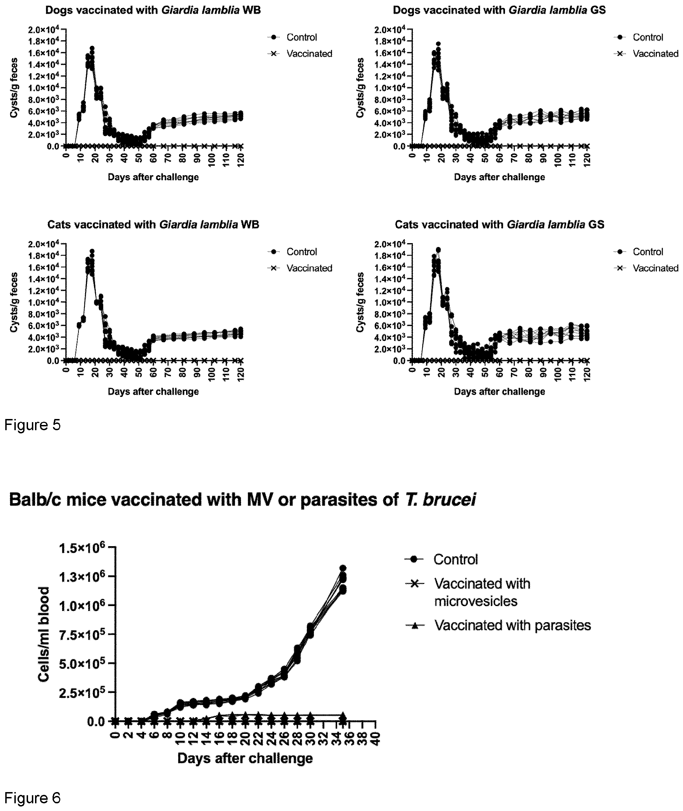

Oral immunization with trophozoites that were previously induced to switch when cultured in the presence of the specific mAb against the corresponding VSP, induced the expression of anti-VSPs slgA for both the A and B assemblage-derived parasites, demonstrating a strong immune response that prevented infection in gerbils and hamsters. In contrast, uninoculated control animals were easily infected by cysts whose VSP was unknown (Figure 4). These same results were observed when vaccinating dogs and cats in the same way (Figure 5).

### Example 2

### 1. T. brucei culture

*T. brucei* in its pleomorphic forms was maintained in an HMI-9 medium supplemented with 10% fetal bovine serum and 10% antibiotic/antimycotic solution at the recommended concentration (Gibco^{®}, Cat. # 15240062). Cell numbers were monitored, diluting them regularly to avoid densities greater than 1.5x106/ml. Pleomorphic forms of the EATRO1125 clone expressing VSG AnTat1.1 were used for switching assays. Fifty parasites expressing a specific VSG (AnTat1.1) were diluted in 20 ml of HMI-9 medium containing 50 nM of the corresponding anti-VSG pAb. They were distributed in 96-well plates. The plates were incubated for 5 days; the number of clones obtained was then counted and the percentage of the original VSGs was measured by IFA. The different clones obtained were amplified in T75 flasks with 30 ml of HMI-9 medium supplemented with 10% fetal bovine serum and 10% antibiotic/antimycotic solution at the recommended concentration (Gibco^{®}, Cat. # 15240062). Cells were labeled with 1/500 polyclonal antisera in a culture medium on ice for 40 min. Subsequently, after two washes with TDB (trypanosome dilution buffer, KCl 5 mM, NaCl 80 mM, MgSO4 1 mM, Na2HPO4 20 mM, NaH2PO4 2 mM, Glucose 20 mM; pH 7. 7), the parasites were fixed in suspension with 4% paraformaldehyde in PBS at room temperature for 1 h. The parasites were then adhered to coverslips using a poly-L-lysine solution. They were washed 3 times with PBS. Blocking was performed with a solution of 3% bovine serum albumin (BSA) in PBS for 1 h. Cells were incubated with Alexa FluorTM-546 goat anti-mouse IgG (H+L) antibody (InvitrogenTM, Cat. # A11030) or Alexa FluorTM-488 goat anti-mouse IgG (H+L) antibody (InvitrogenTM, Cat. # A11003) at 1/1,000 dilutions. For all parasites, nuclei and kinetoplasts were stained with DAPI at a concentration of 1 µg/ml in PBS for 10 min. For IFA, images were acquired using an epifluorescence microscope.

### 2. Laboratory animals

Balb/c mice (6-8 weeks old) of both sexes were used and maintained under specific pathogen-free (SPF) conditions in boxes connected to microisolators, following NIH guidelines for laboratory animals.

### 3. Production of polyclonal antibodies

Polyclonal antisera against *T. brucei* were produced in 6-week-old Balb/c mice. Mice were infected intraperitoneally with 1.5x106 parasites from 4 different clones of pleomorphic forms of EATRO1125 expressing VSG-4156 also called AnTat1.1, VSG-4710, VSG-353 or VSG-62). After 48 h, mice with detectable parasitemia were cured with two consecutive doses, 24 h apart, of 40 mg/Kg BerenilTM (MSD Animal Health^{®}, SAGARPA Reg. Q-0273-121). The entire procedure was repeated 15 days later. Visualization and blood culture of the animals showed the absence of parasites after BerenilTM treatment. At 1 and 2 weeks after the booster (7 and 24 dpi), blood samples were taken from the retro-orbital plexus using heparinized capillaries. From the sera obtained, the presence of antibodies reactive against the surface of the parasites was confirmed by immunofluorescence microscopy (IFA). The serum obtained at 7 dpi was used at 1/1,000 dilution to stimulate switching, while the serum obtained at 24 dpi was used at 1/50 dilution to kill non-switchers.

### 4. Purification of microvesicles

Parasites (2.5x107) were resuspended in 5 ml of HMI-9 medium supplemented with 3% adult bovine serum previously ultracentrifuged overnight at 250,000 g with a 1/50 dilution of polyclonal antibody against the corresponding VSG and cultured 37°C for 30 min in HMI-9 medium. The tubes were then cooled on ice and then centrifuged in an SW41Ti rotor (Beckman) at 4°C for 10 min. The supernatants were collected in 50 ml centrifuge tubes and centrifuged at 3,000 g at 4°C for 40 min; this step was repeated once. The supernatants were then concentrated 10X in a centrifugal filtration device (CentriconTM Plus-70-100K, Millipore^{®}, Cat. # UFC710008) and ultracentrifuged in a Beckman^{®} SW41Ti rotor (25,000 rpm, 2 h, at 4°C). The pellets were washed in an equal amount of ice-cold filtered PBS and ultracentrifuged again to recover the microvesicles. Purification of the microvesicles was validated by IFA.

### 5. Vaccine protection assays in Balb/c mice.

Balb/c mice (6 to 8 weeks old) of both sexes were maintained under specific pathogen-free (SPF) conditions in boxes connected to microisolators, following NIH guidelines for laboratory animals. The animals were free of *T. brucei,* which was determined by blood analysis by immunofluorescence assays using pAbs. Prior to infection, serum from all animals was tested for antibodies to *T. brucei* antigens by enzyme-linked immunosorbent assay using a preparation of total proteins extracted from the parasite. Three days before day 0, animals were weighed and randomly assigned to the experimental groups vaccinated with parasites (n=10), vaccinated with microvesicles (n=10) or unvaccinated control (n=10). Animals were clinically examined weekly throughout the trial. After the animals were infected, blood samples were collected to detect the presence of the parasite.

One group of animals was immunized with two successive intraperitoneal administrations of 300 µL of microvesicles expressing parasite antigens resuspended in sterile PBS/0.01% Tween 20, with an interval of 15 days that were previously induced to switch VSGs by culturing in the presence of the specific pAb against VSG expressed on the surface of the original clone. Another group was immunized with 2×10⁵ parasites resuspended in 300 µL of sterile PBS at a 15-day interval. Parasites were previously induced to switch by culturing in the presence of the specific pAb against VSG that was expressed on the surface of the original clone. The parasites were inactivated by sonication on ice (Al-Sabi MNS et. al., J Appl Microbiol. 2015;119(3):894-903). The absence of live parasites in the preparation was verified by culture in HMI-9 medium for up to 1 week after inoculation and by negativity to fluorescein diacetate staining (Saruyama N, et. al., Anal Biochem [Internet]. 2013;441(1):58-62. Available from: http://dx.doi.org/10.1016/j.ab.2013.06.005). Control animals were inoculated with 300 µL of PBS by the same route.

Sixty days post-immunization all groups were infected via the intraperitoneal route with 103 parasites resuspended in 300 µL of HMI-9 medium. Blood samples were taken from both groups of animals every 2 days between days 0 and 35. The blood sample was treated with 0.8% w/v ClNH₄ for better visualization under microscopy. Pleomorphic parasites were counted in a Neubauer chamber.

### Results

Immunization with microvesicles released after culturing the parasites in the presence of the specific pAb against the initially expressed VSG generated a strong immune response that prevented infection in Balb/c mice. Immunization with inactivated parasites that were previously induced to switch when cultured in the presence of the specific anti-VSG pAb expressed on the surface of the original clone was -70% effective, with infected animals exhibiting low levels of parasitemia in the blood. In contrast, uninoculated control animals were readily infected (Figure 6).

### Example 3

### 1. Tritrichomonas foetus culture

*Trichomonas* parasites were grown axenically at 37°C in TYM medium supplemented with 10% heat-inactivated horse serum (Invitrogen), 10 U penicillin (Invitrogen), 10 g streptomycin (Invitrogen), adjusted to pH 7.0 (Petrin D, et. al., Clin Microbiol Rev. 1998;11(2):300-17). Clones expressing different surface antigens were obtained by limiting dilution in 96-well culture plates at 37°C in TYM medium supplemented with 10% heat-inactivated horse serum (Invitrogen), 10 U penicillin (Invitrogen), 10 mg streptomycin (Invitrogen), adjusted to pH 7.0 for 5 days. Clones were expanded in culture medium overnight. For antigenic switching induction studies, cultures expressing a given surface antigen were cultured in the presence of their specific pAb at the dilution that does not bind the parasites for more than 2 days in 12-well plates. After 5 days of culture, the number of Trichomonas was quantified in a Neubauer chamber and characterization of surface antigen expression was performed by IFA with the pAb corresponding to the original clone.

### 2. Laboratory animals

Balb/c mice (6 to 8 weeks old) of both sexes were used and maintained under specific pathogen-free (SPF) conditions in boxes connected to microisolators, following NIH guidelines for laboratory animals.

### 3. Production of polyclonal antibodies

Polyclonal antisera (pAb) were produced against clones derived from a local isolate of *T. foetus* in 6-week-old Balb/c mice. Mice were infected intraperitoneally with 1.5x106 parasites emulsified with adjuvant (SigmaTM Adjuvant System MPL+TDM+CWS) according to the manufacturer's recommendation. The entire procedure was repeated 21 days later. Ten days after the last immunization, blood samples were taken from the retro-orbital plexus using heparinized capillaries. From the sera obtained, the presence of antibodies reactive against the surface of the parasites was confirmed by immunofluorescence microscopy (IFA). The sera obtained were used at a dilution of 1/1,000 to stimulate switching.

### 4. Vaccine protection assays in Balb/c mice.

Female Balb/c mice (6-8 weeks old) were used and maintained under specific pathogen-free (SPF) conditions in boxes connected to microisolators, following NIH guidelines for laboratory animals. The animals were free of *Tritrichomonas foetus,* which was determined by blood analysis by immunofluorescence assays using pAbs. Prior to infection, serum from all animals was tested for antibodies to T. foetus antigens by enzyme-linked immunosorbent assay using a preparation of total proteins extracted from the parasite. Three days before day 0, animals were weighed and randomly assigned to the experimental parasite-vaccinated (n=10) or control (n=10) groups. Animals were clinically examined weekly throughout the trial. After the animals were infected, blood samples were collected to detect the presence of the parasite.

One group of animals was immunized with two successive oral administrations of 300 µL of *Tritrichomonas foetus* (2×10⁶ trophozoites per kilogram body weight) expressing different parasite antigens resuspended in sterile PBS/0.01% Tween 20, with an interval of 15 days. *T. foetus* cells were from a clonal population and were induced to switch their surface antigen when cultured in the presence of the original clone-specific pAb. *Tritrichomonas* were inactivated by sonication on ice (Al-Sabi MNS et. al., J Appl Microbiol. 2015;119(3):894-903). The absence of live parasites in the preparation was verified by culture in TYM medium supplemented with 10% heat-inactivated horse serum (Invitrogen), 10 U penicillin (Invitrogen), 10 mg streptomycin (Invitrogen), adjusted to pH 7.0 for up to one-week post inoculation and by negativity to fluorescein diacetate staining (Saruyama N, et. al., Anal Biochem [Internet]. 2013;441(1):58-62. Available from: http://dx.doi.org/10.1016/j.ab.2013.06.005). Control animals were inoculated with 300 µL of PBS by the same route.

Twelve days after inoculation, vaginal cytology was analyzed in both infected and control groups to determine the efficacy of immunization.

### Results

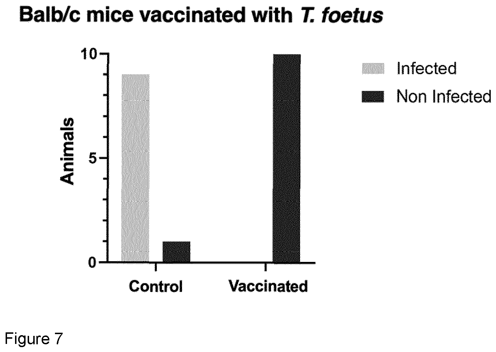

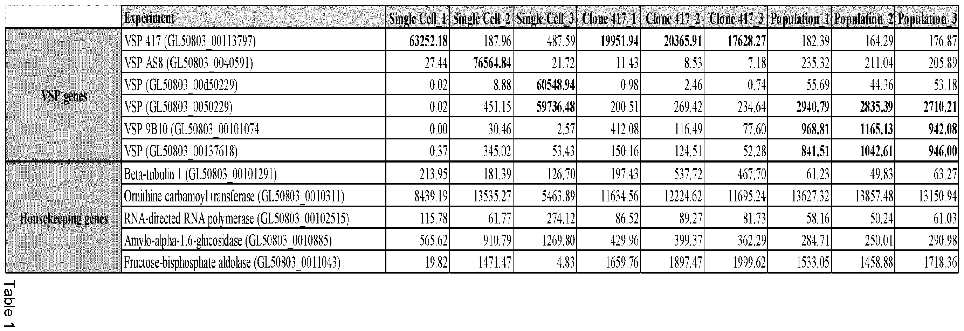

It was observed that when a clonal population of Tritrichomonas was cultured in 12-well plates in the continuous presence of its corresponding pAb (50 nM), after 5 days, in each well the expression of the surface antigen was modified, expressing in all cases antigens different from the original one, but not in the control experiment, where the rate of variation never exceeded 15% in that given period (Table 3). Immunization with T. foetus after culturing them in the presence of the specific pAb against the initially expressed surface antigen generated a strong immune response that prevented infection in Balb/c mice. In contrast, non-inoculated control animals were easily infected (90%) (Figure 7).

**Table 3. Antibody-induced antigenic variation. Percentage of switchers after culturing a clonal population of Tritrichomonas with their respective specific pAb and without antibody.**

| | | mAb | | | Control | | |
|---|---|---|---|---|---|---|---|
| Culture # | Well # | Clon1 Switch ers | Clon2 Switch ers | Clon3 Switch ers | Clon1 Switch ers | Clon2 Switch ers | Clon3 Switch ers |
| 1 | 1 | 97% | 100% | 98% | 0% | 0% | 0% |
| | 2 | 97% | 99% | 99% | 5% | 1% | 3% |
| | 3 | 98% | 99% | 97% | 12% | 3% | 0% |
| | 4 | 99% | 97% | 99% | 7% | 6% | 0% |
| | 5 | 96% | 99% | 100% | 4% | 0% | 3% |
| | 6 | 97% | 100% | 100% | 2% | 1% | 6% |
| | 7 | 98% | 97% | 96% | 7% | 1% | 11% |
| | 8 | 97% | 98% | 97% | 0% | 10% | 3% |
| | 9 | 99% | 96% | 98% | 0% | 4% | 0% |
| | 10 | 98% | 99% | 100% | 3% | 2% | 8% |
| | 11 | 100% | 96% | 100% | 6% | 0% | 0% |
| | 12 | 97% | 97% | 97% | 9% | 6% | 4% |
| | | 99% | 100% | 98% | 14% | 8% | 2% |
| | | 99% | 99% | 97% | 0% | 11% | 0% |
| | | 98% | 98% | 98% | 2% | 0% | 0% |
| | | 98% | 98% | 96% | 4% | 3% | 5% |
| | | 97% | 97% | 97% | 6% | 0% | 0% |
| | | 99% | 98% | 99% | 0% | 0% | 2% |
| | | 98% | 100% | 98% | 0% | 0% | 6% |
| | | 100% | 99% | 98% | 1% | 1% | 3% |
| | | 99% | 98% | 100% | 15% | 3% | 8% |
| | | 97% | 100% | 99% | 6% | 0% | 1% |
| | | 99% | 1% | 97% | 0% | 1% | 0% |
| | | 98% | 98% | 98% | 0% | 0% | 0% |

## Claims

1. Vaccines for preventing or treating protozoan parasite infections in an individual said vaccine comprising an active agent, and excipients, diluents or carriers and, optionally, adjuvants, wherein the active agent comprises multiple variable surface antigens (VSAs) of the parasite to be treated or to be protecting against whereby the multitude of VSAs of the parasites are obtained by induction of expressing of multiple VSAs in a parasite clone by inducing the switching of the variant surface antigen of said clone expressing a single VSA whereby said clone was grown in the presence of a specific antibody against the single VSA present on the surface of said clone to induce the switching of this VSA and the expression of multiple distinguishable VSAs in the population obtained by said culturing the clone.

2. The vaccine according to claim 1 further containing microvesicles released by said treatment containing a variety of protozoan antigens.

3. The vaccine according to claim 1 or 2 being a protective vaccine against subsequent infection with the protozoan parasite.

4. The vaccine according to any one of the preceding claims, wherein the protozoan parasite undergoes antigenic variation, which examples are selected from the genus of *Trypanosoma, Trichomonas, Tritrichomonas, Giardia.*

5. The vaccine according to claim 4, wherein the protozoan parasite is selected from *Trypanosoma brucei, Trichomonas vaginalis, Tritrichomonas foetus, Giardia lamblia.*

6. The vaccine according to any one of the preceding claims, wherein the individual is a mammal like a domestic or farm animal, or a human.

7. The vaccine according to any one of the preceding claims, wherein the active agent is provided in a form selected from: attenuated parasites, death parasites, parts of the parasites including plasma membrane, purified VSAs, microvesicles containing parasite antigens, or combinations thereof.

8. The vaccine according to any one of the preceding claims for mucosal and/or systemic administration.

9. The vaccine according to claim 8, wherein it is administered subcutaneously or intramuscularly.

10. The vaccine according to any one of the preceding claims comprising administration of two doses of VSAs in an amount of 50 to 500, like 200 µg per g body weight within an interval of 10 to 20 days, like 15 days.

11. The vaccine according to any one of claims 1 to 10, wherein the vaccine is provided in a formulation of a topical creme for administering multiple times, in particular, 15 days for two months.

12. The vaccine according to any one of the preceding claims being a protective vaccine for administration by food or drinking water of the mammal to be treated.

13. A method for producing a vaccine according to any one of claims 1 to 12 comprising the steps of:
- Providing a protozoan parasite clone expressing a single type of multiple variable surface antigens (VSAs) encoded in the genome of said protozoan parasite clone;
- Culturing said protozoan parasite clone in the presence of a specific antibody against the single type of VSA for inducing the switching of VSA;
- Propagation of the cultured and switched protozoan parasite expressing multiple VSAs;
- Obtaining multiple VSAs in a form applicable in a vaccine;
- Optionally, obtaining microvesicles in a form applicable in a vaccine; and
- Preparing the vaccine including mixing with excipients, diluents or carriers.

14. The method for producing the vaccine according to claim 13 wherein the specific antibody against the single type of VSA is a monoclonal antibody.

15. The method for producing the vaccine according to claim 13 or 14 wherein the propagation is conducted in the presence of the specific antibody directed to the expressed VSA, preferably the specific antibody can be a monoclonal antibody or polyclonal antibodies. Like being generated in mice, rats, rabbits, or goats.

16. The method for producing the vaccine according to any one of claims 13 to 15 wherein the step of obtaining the multiple VSA in a form applicable in a vaccine includes the purification and/or the inactivation of the protozoan parasite after cultivation and propagation.

17. The method for producing the vaccine according to any one of claims 13 to 16 wherein microvesicles containing a variety of protozoan antigens when obtained through release by said treatment are purified from the culture supernatant by centrifugation and filtration.
